Europäisches Patentamt

European Patent Office

Office européen des brevets

(11) Veröffentlichungsnummer: **0 321 389**
**A1**

# (12) EUROPÄISCHE PATENTANMELDUNG

(21) Anmeldenummer: **88730279.2**

(22) Anmeldetag: **14.12.88**

(51) Int. Cl.4: **A 61 F 2/30**

(30) Priorität: **14.12.87 DE 8716607**

(43) Veröffentlichungstag der Anmeldung:
**21.06.89 Patentblatt 89/25**

(84) Benannte Vertragsstaaten:
**AT BE CH DE ES FR GB GR IT LI NL SE**

(71) Anmelder: **MECRON medizinische Produkte GmbH**
**Nunsdorfer Ring 23-29**
**D-1000 Berlin 48 (DE)**

(72) Erfinder: **Gross, Ulrich, Prof.Dr.**
**Gelfertstrasse 17**
**D-1000 Berlin 33 (DE)**

**Schmitz, Herman-Josef, Dr.Dr.**
**Fronhofer Strasse 14**
**D-1000 Berlin 41 (DE)**

**Kranz, Curt, Dr.**
**Kufsteiner Strasse 12**
**D-1000 Berlin 62 (DE)**

(74) Vertreter: **Christiansen, Henning, Dipl.-Ing.**
**Patentanwalt CHRISTIANSEN Pacelliallee 43/45**
**D-1000 Berlin 33 (DE)**

(54) Implantierbare Prothese.

(57) Implantierbare Prothese bestehend aus einem Prothesenkopf und einem damit verbundenen Prothesenschaft und einer den Prothesenschaft umgebenden Prothesenverankerung aus einem bioaktiven, resorbierbaren, poröse keramische Bestandteile enthaltenden Werkstoff, wobei die Prothesenverankerung (3) mit einem Außengewinde (30) versehen ist, wobei das Außengewinde (30) aus einem verrundeten oder Rundgewinde besteht.

FIG. 1

EP 0 321 389 A1

## Beschreibung

### Implantierbare Prothese

Die Erfindung betrifft eine implantierbare Prothese der im Oberbegriff des Anspruchs 1 angegebenen Art.

Aus der DE-C-26 20 907 ist eine implantierbare Prothesenverankerung bekannt, die aus bioaktiven resorbierbaren ke ramischen Werkstoffen auf der Basis von Kalziumphosphaten sowie aus polymeren Kunststoffen besteht, die im Körpermilieu mechanisch und chemisch stabil sind. Mit einer derartigen Prothesen Verankerung werden wesentliche Nachteile einer mit Hilfe eines erhärtenden Knochenzements befestigten metallischen Prothese beseitigt, deren Prothesenschaft in eine entsprechend herausgearbeitete Höhlung im Knochen mechanisch verankert wird. Diese Art der Sekundärfixation löst jedoch aufgrund der polymerisierenden Zementbestandteile schädliche Nebenreaktionen im Organismus aus, die zur Gewebezerstörung und damit zu einer Lockerung der Prothese führen.

Die aus der DE-C-26 20 907 bekannte Prothesenverankerung ist als Prothesenschaftbeschichtung ausgebildet und weist keramische Werkstoffe in partikulärer Form auf, die in ei nem polymeren Kunststoff eingelagert sind, so daß bei der Resorption des keramischen Anteils der Prothesenverankerung ein durchgängig poröses Gefüge aus Kunststoff entsteht, auf dessen inneren Porenoberflächen bioaktivierende Reste der Keramik zurückbleiben. Auf diese Weise soll eine bindegewebsfreie Verwachsung des Knochens mit der Werkstoffoberfläche bewirkt werden, so daß ein auch für hochbelastete Endoprothesen geeignetes Implantat geschaffen wird, das eine dauerhafte Verbndung mit dem Knochengewebe einerseits und mit der Endoprothese bzw. dem prothesenschaft andererseits sicherstellt. Die feste Verbindung der Prothesenverankerung mit dem Knochengewebe wird dadurch erzielt, daß nach der Implantation neu gebildetes Knochengewebe simultan zu der Porenbildung bzw. zu der Resorption der Keramik in das poröse Kunststoffgefüge penetrieren kann.

Da die bekannte Prothesenverankerung als Prothesenschaftbeschichtung ausgebildet ist und im wesentlichen kraftschlüssig im Implantatlager des Knochens verankert wird, ist einerseits eine nur dünne Schicht bioaktiven, resorbierbaren, porösen keramischen Werkstoffes zum Einwachsen von Knochengewebe gegeben und andererseits ein sehr präzises Herausarbeiten der Höhlung im Knochen zur Schaffung des Implantatlagers erforderlich. Die geringe Schicht bioaktiven, keramischen Werkstofes in Verbndung mit einer kleinen, der Außenfläche des Prothesenschaftes entsprechenden Oberfläche sowie aufgrund der rein kraftschlüssigen Verbindung ist eine erhebliche Zeit zum Einwachsen von Knochengewebe in die Prothesenverankerung erforderlich und es besteht darüber hinaus die Gefahr, daß sich die Prothese trotz Verwendung eines porösen keramischen Materials lockert.

Aus der EP-BI-0 083 028 ist ein Verbindungselement für einen mit einer Bohrung versehenden Implantatkörper bekannt, der aus einem biokompatiblen oder bioaktiven Material besteht. Es weist eine Innenbohrung oder ein Innengewinde zur Aufnahme des Verbindungselementes auf, das aus einem Material hoher Biegefestigkeit besteht, dessen Durchmesser kleiner als derjenige der Bohrung ist und der in dem Hohlraum zwischen dem Stift und der Wand des Implantatkörpers mit diesem in Verbindung steht. Zusätzlich ist der Stift mit einer kugel-, tonnen- oder zylinderförmig ausgebildeten Verdickung versehen, die an der Bohrung des Implantats anliegt und eine entsprechende Fixierung des Stiftes im Implantat bewirkt. Der bekannte Implantatkörper wird jedoch analog zur vorstehend beschriebenen Prothesenveran kerung lediglich in einer entsprechend herausgearbeiteten Höhlung eingesetzt bzw. verankert, so daß insbesondere bei hochbelasteten Endoprothesen die Gefahr einer Lockerung der Prothese nach wie vor besteht.

Weiterhin ist aus der DE-A-27 17 506 ein keramisches Knochenimplantat bekannt, das aus einem Kernkörper und einer Beschichtung besteht, das mit einer resorbierbaren mikroporösen Struktur versehen ist. Das äußere Element weist ein Spitzgewinde auf.

Hierbei besteht der Nachteil, daß mit der Zeit eine Lockerung eintritt.

Der Erfindung liegt die Aufgabe zugrunde, eine implantierbare Prothese der eingangs genannten Gattung zu schaffen, die auch für hochbelastete Endoprothesen geeignet ist und eine dauerhafte Verbindung zwischen dem Knochengewebe und der Prothesenverankerung einerseits sowie der Prothesenverankerung und dem Prothesenschaft andererseits gewährleistet.

Diese Aufgabe wird mit dem kennzeichnenden Merkmal des Anspruchs 1 gelöst

Bei der erfindungsgemäßen Lösung wurde von der Erkenntnis ausgegangen, daß die Vorteile eines bioaktiven, resorbierbaren, porösen keramischen Werkstoffes in Bezug auf die Gewebeverträglichkeit und eine direkte, bindegewebsfreie Verwachsung des Knochens mit dem Werkstoff in Verbindung mit einer entsprechenden Formgebung der Außenfläche der Prothesenverankerung zu einer optimalen Befestigung der Prothese im Implantatlager führt. Die optimale Befestigung wird durch eine kraft- und formschlüssige Verbindung der Prothesenverankerung mit dem Implantatlager erzielt, wofür ein Rundgewinde die besten Voraussetzungen schafft. Zur Befestigung der Prothesenverankerung im Implantatlager wird ein entsprechendes Gewinde in das Implantatlager geschnitten und die Prothese, deren Verankerung ein entsprechendes Gewinde aufweist, in das Innengewinde des Implantatlagers eingeschraubt.

Die Erfindung beruht dabei auf der Erkenntnis, daß bei einem Rundgewinde die örtlichen Spannungsspitzen in den aneinander angrenzenden Materialien ein Minimum sind, so daß eine maximale Festigkeit der Verbindung auch bei Schubbeanspruchungen gewährleistet ist.

Auf diese Weise wird bereits unmittelbar nach der Implantation ein fester Sitz erzielt und darüber hinaus aufgrund der vergrößerten Oberfläche eine große Fläche zum Einwachsen von Knochengewebe bereitgestellt.

In einer vorteilhaften Weiterbildung der Erfindung besteht das Außengewinde aus einem Rundgewinde, das vom Prothesenkopf zum Ende des Prothesenschaftes hin leicht konisch verlaufend ausgebildet ist. Das Rundgewinde ist nach Art eines Glühlampengewindes ausgebildet, so daß keine scharfen Kanten vorhanden sind, die zu Reizungen oder Überbeanspruchungen auch des Knochengewebes führen könnten.

In einer weiteren vorteilhaften Ausgestaltung der Erfindung ist der Prothesenschaft kegelstumpfförmig ausgebildet und weist Einkerbungen zur Aufnahme des Verankerungswerkstoffes auf. Dabei sind vorteilhafter Weise jeweils zwei Einkerbungen parallel zueinander und orthogonal zur Mittellängsachse des Prothesenschaftes verlaufend angeordnet. Auf diese Weise wird eine feste Verbindung zwischen Prothesenschaft und Prothesenverankerung gewährleistet, die auch beim Eindrehen der Prothese in das Implantatlager nicht gelöst wird.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:

Figur 1 einen Querschnitt durch die erfindungsgemäße implantierbare Prothese und

Figur 2 einen Schnitt durch die in Figur 1 dargestellte Prothese entlang der Linie A-A.

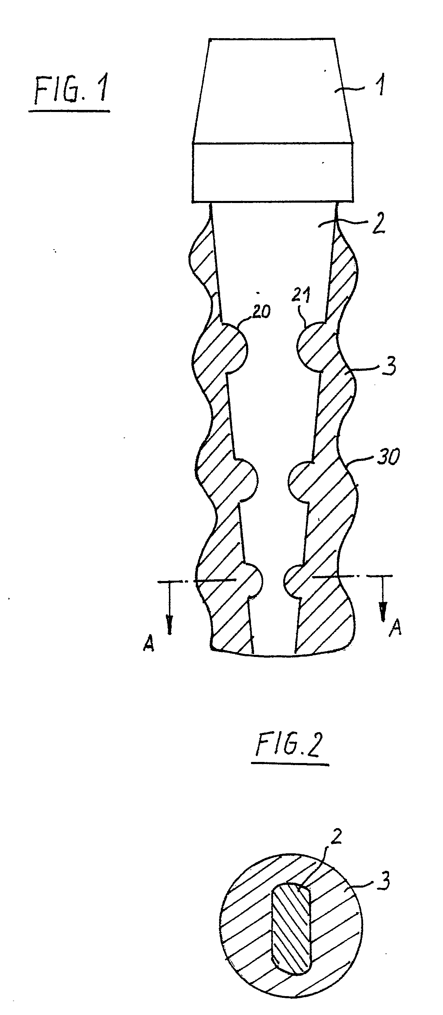

In Figur 1 ist ein Längsschnitt durch die erfindungsgemäße implantierbare Prothese dargestellt, die als Zahnimplantat Verwendung findet und aus einem Prothesenkopf 1, einem Prothesenschaft 2 und einer Prothesenverankerung 3 besteht. Der Prothesenkopf 1 und der Prothesenschaft 2 bestehen wahlweise aus Metall- oder Oxidkeramik, vorzugsweise aus Titan oder einer Titanlegierung. Anstelle des in Figur 1 dargestellten Prothesenkopfes kann auch ein einer Gelenkkugel nachgebildeter Prothesenkopf für beispielsweise eine Hüftgelenkprothese vorgesehen sein.

Die Prothesenverankerung 3 besteht aus einem bioaktiven, resorbierbaren, keramischen, porösen Werkstoff, nämlich einem auf Keramik gesinterten Keramikmaterial, das unter der Bezeichnung "Hot-Isostatic-Pressurized"(HIP)-Material erhältlich ist. Dieses ist beispielsweise in der DE-A-36 15 732 näher beschrieben.

Die Porosität des Materials wird vorzugsweise durch Abtragen von Material hergestellt. Der keramische Werkstoff ist dabei so beschaffen, daß nach der Implantation neu gebildetes Knochengewebe in das poröse Gefüge eindringt, wobei die bioaktivierende Keramik ein Eindringen des Knochengewebes in die Oberfläche fördert. Infolge des bioaktivierenden Materials wird die Ausbildung einer bindegewebigen Zwischenschicht, die als Hauptursache für die Lockerung von Implantaten angesehen wird, verhindert.

Zur Vergrößerung der Oberfläche der Prothesenverankerung sowie zur form- und kraftschlüssigen Verbindung der Prothesenverankerung mit dem Implantatlager ist auf der Außenfläche der Prothesenverankerung 3 ein Außengewinde 30 in Form eines Rundgewindes vorgesehen. Die Prothesenverankerung 3 verläuft vorzugsweise vom Prothesenkopf 1 zum Ende des Prothesenschaftes 2 hin leicht konisch. Diese Ausgestaltung des Gewindes verhindert Spannungsspitzen im Material und damit eine örtliche Überbeanspruchung der Grenzschicht Prothese/Knochenmaterial, so daß auf diese Weise ein dauerhafter Sitz der Prothese gewährleistet ist. Spannungsspitzen würden sonst zu Rissen führen, welche - da sie Materialschwächungen bilden - wiederum zur Überbeanspruchung benachbarter Materialbereiche und damit zur Fortsetzung der Rißbildung bis zur totalen Lockerung der Prothese führen würden.

Zur Befestigung der implantierbaren Prothese im Implantatlager wird ein entsprechendes Rundgewinde als Innengewinde in das Implantatlager geschnitten und daran anschließend die Prothese mit der Prothesenverankerung in das Implantatlager eingeschraubt. Daran anschließend wird beispielsweise bei einer Hüftgelenkprothese eine dem Kugelgelenk des Hüftgelenks entsprechende Kugel auf dem Prothesenkopf 1 ausgerichtet und in geeigneter Weise befestigt.

Der Prothesenschaft 2 ist kegelstumpfförmig ausgebildet und weist über seine Länge verteilt angeordnete Einkerbungen 20, 21 auf, wobei jeweils zwei Einkerbungen 20, 21 parallel zueinander und orthogonal zur Mittellängsachse des Prothesenschaftes 2 verlaufen (vgl. Figur 2).

Die Anzahl und der Abstand der Einkerbungen 20, 21 wird durch die Art der jeweiligen Prothese bestimmt, wobei beispielsweise bei Endoprothesen 3 bis 4 Einkerbungs-Paare 20, 21 ausreichend sind.

In einer alternativen Ausführungsform kann der Prothesenschaft 2 anstelle von Einkerbungen mit einem Außengewinde versehen werden, das dieselbe Gangrichtung aufweist wie die Gangrichtung des Außengewindes der Prothesenverankerung 3. Auf diese Weise wird verhindert, daß beim Eindrehen der Prothese in das Implantatlager eine Lockerung zwischen Prothesenschaft 2 und Prothesenverankerung 3 auftritt, da die tragenden Bereiche der Zwischenschicht maximale Abmessungen aufweisen.

Die Einkerbungen 20, 21 bzw. die Gewindeverbindung zwischen Prothesenschaft 2 und Prothesenverankerung 3 dienen zur Herstellung einer formschlüssigen Verbindung zwischen Prothesenschaft 2 und Prothesenverankerung 3 zur Aufnahme hoher Zug- und Druck- sowie Drehbelastungen.

Die Erfindung beschränkt sich in ihrer Ausführung nicht auf das vorstehend angegebene bevorzugte Ausführungsbeispiel. Vielmehr ist eine Anzahl von Varianten denkbar, welche von der dargestellten Lösung auch bei grundsätzlich anders gearteten Ausführungen Gebrauch machen.

## Patentansprüche

1. Implantierbare Prothese bestehend aus einem Prothesenkopf und einem damit verbundenen Prothesenschaft und einer den Prothesenschaft umgebenden Prothesenverankerung aus einem bioaktiven, resorbierbaren, poröse keramische Bestandteile enthaltenden Werkstoff, wobei die Prothesenverankerung (3) mit einem Außengewinde (30) versehen ist, **dadurch gekennzeichnet,** daß das Außengewinde (30) aus einem verrundeten oder Rundgewinde besteht.

2. Implantierbare Prothese nach Anspruch 1, **dadurch gekennzeichnet,** daß es sich bei dem Werkstoff der Prothesenverankerung um HIP-Material handelt und der Prothesenschaft bzw. -kopf aus einer Titanlegierung besteht.

3. Implantierbare Prothese nach Anspruch 1 oder 2, **dadurch gekennzeichnet,** daß die Prothesenverankerung (3) vom Prothesenkopf (1) zum Ende des Prothesenschaftes (2) konisch verlaufend ausgebildet ist.

4. Implantierbare Prothese nach einem der vorangehenden Ansprüche, **dadurch gekenn-** **zeichnet,** daß die Prothesenverankerung (3) an ihrem dem Prothesenkopf entgegengesetzten Ende abgerundet ist.

5. Implantierbare Prothese nach einem der vorangehenden Ansprüche, **dadurch gekenn-** **zeichnet,** daß der Prothesenschaft (2) kegelstumpfförmig ausgebildet ist und über sein Länge verteilt mehrere Einkerbungen (20, 21) zur Aufnahme des Verankerungswerkstoffes aufweist.

6. Implantierbare Prothese nach Anspruch 5, **dadurch gekennzeichnet,** daß jeweils zwei Einkerbungen (20, 21) parallel zueinander und orthogonal zur Mittelängsachse des Prothesenschaftes (2) verlaufen.

7. Implantierbare Prothese nach Anspruch 5 oder 6, **dadurch gekennzeichnet,** daß die Einkerbungen (20, 21) über die Länge des Prothesenschaftes (2) vom Prothesenkopf (1) zum Ende des Prothesenschaftes (2) einen abnehmenden Durchmesser aufweisen.

8. Implantierbare Prothese nach einem der vorangehenden Ansprüche, **dadurch gekenn-** **zeichnet,** daß der Prothesenschaft (2) mit einem Außengewinde versehen ist, das dieselbe Gangrichtung wie das Außengewinde (30) der Prothesenverankerung (3) aufweist.

FIG. 1

FIG.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | GB-A-2 162 753 (CHAS F. THACKRAY LTD) * Seite 1, Zeilen 81-87; Seite 3, Zeilen 1-6; Figur 4 * | 1-8 | A 61 F 2/30 |
| P,Y | FR-A-2 610 512 (CUILLERON) * Figur 5 * | 1-8 | |
| D,Y | WO-A-8 706 842 (DEUTSCHER) * Seite 1, Zeilen 1-5; Seite 16, Zeilen 13-21 * | 2 | |
| D,Y | DE-A-2 717 506 (KYOTO) * Seite 23, Zeilen 29-32; Figur 11; Seite 22, Zeilen 2-6; Figur 5 * | 5-8 | |

**RECHERCHIERTE SACHGEBIETE (Int. Cl.4)**

A 61 F
A 61 C

Der vorliegende Recherchenbericht wurde für alle Patentansprüche erstellt

| Recherchenort | Abschlußdatum der Recherche | Prüfer |
|---|---|---|
| DEN HAAG | 10-03-1989 | MOERS R.J. |